# FASCICULE DE BREVET EUROPEEN

(11) **EP 4 299 039 B1**
(45) Date de publication et mention de la délivrance du brevet: **01.04.2026**
(21) Numéro de dépôt: 23315251.1
(22) Date de dépôt: 19.06.2023
(51) Int. Cl.: A61F 2/44

(54) **CAGE INTERVERTÉBRALE**
ZWISCHENWIRBELKÄFIG
INTERVERTEBRAL CAGE

(30) Priorité: 28.06.2022 FR 2206418
(43) Date de publication de la demande: 03.01.2024
(73) Titulaire: Razian, Hassan, 94240 L'Hay-les-Roses (FR)
(72) Inventeur: Razian, Hassan, 94240 L'HAY-les-ROSES (FR); Razian, Salar, 94240 L'HAY-les-ROSES (FR)
(74) Mandataire: Boüan du Chef du Bos, Louis-Paterne

(56) Documents cités:
- WO-A1-2021/162754
- FR-A1- 2 955 483
- FR-A1- 2 965 169
- US-A1- 2011 035 007

## Description

### Domaine technique

La présente invention concerne les cages intersomatiques aptes à être interposées entre deux vertèbres en remplacement du disque intervertébral originellement situé entre ces deux vertèbres, du type comprenant un élément présentant la forme générale d'un disque sensiblement cylindrique et des moyens de blocage de cet élément avec au moins l'une des deux vertèbres afin d'obtenir la fixation des cages par ostéosynthèse.

Il est connu de telles cages intersomatiques dans lesquelles les moyens de blocage du disque avec au moins l'une des deux vertèbres entre lesquelles il est mis en place, sont constitués par une ou deux lames aptes à pivoter pour que leurs extrémités se plantent dans l'élément osseux des vertèbres. Une telle réalisation à deux lames parallèles et sensiblement perpendiculaires aux plans des faces opposées du disque est par exemple décrite et illustrée dans le FR2965169, le US2011/035007, le FR3029770 et le FR2955483. WO 2021/162754 A1 décrit un implant destiné à un joint ou à une cavité créée chirurgicalement, comportant une lame présentant une torsion hélicoïdale.

La cage intersomatique selon le document référencé ci-dessus donne de bons résultats, mais peut parfois ne pas répondre à tous les besoins des Praticiens en fonction notamment de différentes pathologies qui affectent les patients qu'ils ont à soigner, les Praticiens éprouvant en plus, dans certains cas, des difficultés à faire pénétrer les lames dans l'os cortical des vertèbres.

La présente invention a pour but de réaliser une cage intersomatique qui pallie en grande partie les inconvénients mentionnés ci-dessus des cages de l'art antérieur.

Plus précisément, la présente invention a pour objet une cage intersomatique apte à être interposée entre deux corps de vertèbres en remplacement du disque intervertébral originellement situé entre ces deux vertèbres selon au moins l'une des revendications annexées

D'autres caractéristiques et avantages de l'invention apparaîtront au cours de la description suivante donnée en regard des dessins annexés à titre illustratif mais nullement limitatif, dans lesquels :
La [Fig. 1] représente une vue en coupe schématique d'un mode de réalisation de la cage intersomatique selon l'invention,
La [Fig. 2] représente une vue en coupe schématique d'un autre mode de réalisation de la cage intersomatique selon l'invention,
La [Fig. 3] représente une vue en perspective cavalière d'un autre mode de réalisation de la cage intersomatique.

Il est précisé que, dans la présente description, si l'adverbe "sensiblement" est associé à un qualificatif d'un moyen donné, ce qualificatif doit être compris au sens strict ou approché.

La présente invention concerne une cage intersomatique apte à être interposée entre deux corps de vertèbres en remplacement du disque intervertébral originellement situé entre ces deux vertèbres, disque qui est endommagé ou le siège d'une maladie.

Ces cas sont bien connus des Praticiens et ne seront pas plus amplement développés ici, d'autant plus qu'ils n'entrent pas dans le cadre de la présente invention.

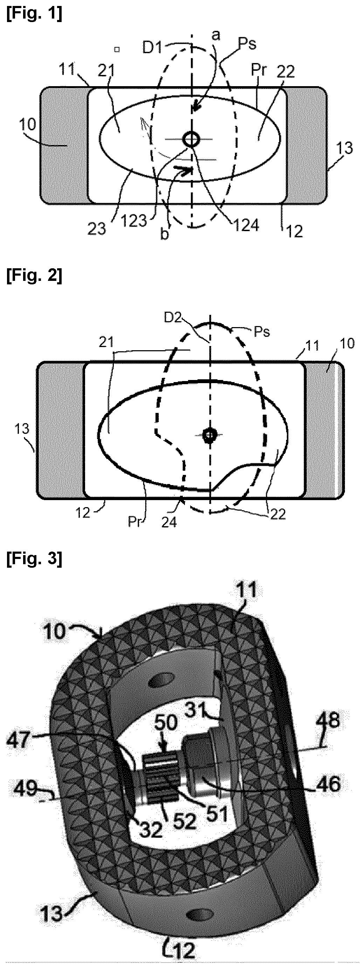

Une telle cage comprend un élément présentant la forme générale d'un disque 10, figures 1-3. Ce disque comprend deux faces opposées 11, 12 sensiblement parallèles et une paroi latérale 13 reliant les deux faces opposées, les deux faces opposées étant aptes à venir au contact respectivement des deux corps vertébraux et définissant sensiblement deux plans dits « plans faciaux » contenant ces deux faces. Ces plans faciaux sont considérés comme parallèles, ou assimilables à deux plans parallèles, car les deux faces 11, 12 peuvent être strictement parallèles ou présenter une très légère angulation l'une par rapport à l'autre suivant les cas de la thérapie préconisée par le Praticien.

La cage comporte en outre des moyens de blocage de l'élément 10 avec au moins l'un des deux corps vertébraux, avantageusement les deux. Ces moyens de blocage comportent au moins une première portion de lame 21 et une seconde portion de lame 22, étant précisé, comme il sera explicité ci-après, que ces deux portions peuvent appartenir à une seule lame selon l'invention, ou à deux lames différentes, selon un mode de réalisation non revendiqué.

La cage comporte donc, comme les cages de l'art antérieur, des moyens pour commander en déplacement ces première et seconde portions de lame entre deux positions Pr, Ps. La première position Pr est celle dans laquelle les première et seconde portions de lame 21, 22 sont entièrement contenues dans l'espace défini entre les deux plans faciaux, et la seconde position Ps est celle dans laquelle ces première et seconde portions de lame 21, 22 émergent au moins partiellement de cet espace.

Selon une caractéristique essentielle de l'invention, les moyens pour commander en déplacement les première et seconde portions de lame 21, 22 entre la première position Pr et la seconde position Ps sont agencés pour commander le déplacement de ces première et seconde portions de lame selon un déphasage non nul de façon que l'une des première et seconde portions de lame émerge, au moins partiellement de l'espace entre les deux plans faciaux, avant l'autre portion de lame.

Selon une autre caractéristique de l'invention, le déphasage en déplacement de ces première et seconde portions de lame 21, 22 entre les deux positions Pr, Ps est l'un des trois déphasages suivants : déphasage spatial, déphasage temporel, une combinaison de ces déphasages spatial et temporel.

Comme mentionné ci-avant, ces première et seconde portions de lame 21, 22 peuvent être définies sur une seule lame 23, 24 (figures 1 et 2).

Dans le mode de réalisation selon la figure 1, cette seule lame 23 est symétrique par rapport à une première droite D1, et les moyens pour commander en déplacement les première et seconde portions de cette lame 23, à savoir les extrémités de la lame, comportent : un arbre de rotation 123 d'axe de rotation 124 et des moyens pour solidariser la lame 23 sur l'arbre 123. Cet arbre est monté en coopération par rapport à l'élément 10 de façon que son axe de rotation 124 soit avantageusement sensiblement parallèle aux faces opposées 11, 12, mais en étant excentré par rapport à ces deux faces, c'est-à-dire de façon qu'il soit situé à une distance différente de ces deux faces. Dans le mode de réalisation selon la figure 1, l'axe de rotation 124 est plus près de la face 11 que de la face 12 (comme illustré par les deux valeurs de distance **a** < **b**).

Dans le mode de réalisation selon la figure 2, la seule lame 24 est asymétrique par rapport à une seconde droite D2. Dans cette réalisation de cage faisant application de l'invention comme définie ci-avant, les deux portions de lame 21, 22 sont définies à chaque extrémité de l'unique lame 24 présentant une asymétrie par rapport à cette seconde droite D2. Dans ce cas, l'axe de rotation 124 défini ci-avant peut être situé à une même distance des deux faces, mais il serait possible qu'il soit excentré par rapport à ces deux faces.

Dans le mode de réalisation (non revendiqué) selon la figure 3, les deux portions de lame 21, 22 sont définies sur respectivement deux lames différentes, à savoir une première lame 31 et une seconde lame 32.

Dans ce mode de réalisation, les moyens pour commander en déplacement la première portion de lame et la seconde portion de lame comportent : des premier et second arbres 46, 47 montés rotatifs par rapport à l'élément 10 autour respectivement d'un premier axe 48 et d'un second axe 49 ; des moyens pour solidariser les première et seconde lames 31, 32 avec respectivement ces premier et second arbres 46, 47, par exemple par réalisation d'une seule pièce ou par soudage ou analogue, de façon que leurs plans fassent un angle déterminé par rapport aux premier et second axes (par exemple 90°, ou moins mais non nul) ; et des moyens pour entraîner en rotation ces premier et second arbres, par exemple un engrenage d'entraînement 50 monté en coopération avec respectivement ces premier et second arbres.

Cet engrenage est constitué par un système mécanique composé d'au moins une première et une deuxième roues dentées (ou analogues) 51, 52 engrenées l'une dans l'autre (ou entraînées par exemple par une courroie sans glissement), les deux roues dentées étant respectivement montées en coopération de fixation avec les arbres de rotation 46, 47 ; et de moyens 35 pour commander la rotation d'au moins l'un des premier et second arbres.

Très avantageusement, au moins l'une des deux roues dentées 51, 52 est montée de façon amovible sur son arbre, pour pouvoir être changée si besoin. Il est aussi possible que l'ensemble comprenant les lames, arbres, ..., soit monté de façon amovible par rapport à l'élément 10 de façon à pouvoir, si nécessaire, remplacer tout l'ensemble.

Le fonctionnement d'une telle cage et ses avantages apparaissent de façon évidente à la description qui en a été faite ci-dessus.

Ils peuvent cependant se résumer de la façon suivante. La cage est introduite entre deux vertèbres, avec la ou les lames orientées de façon que leurs portions 21, 22 soient dans la première position Pr. Cette technique est largement connue des Praticiens. Puis la ou les lames sont pivotées, à l'aide d'un ancillaire adapté connu en lui-même. Comme les portions de lame sont déphasées dans leur rotation, c'est en premier lieu l'une des deux, par exemple la première portion de lame 22, qui pénètre dans l'os cortical de l'une des vertèbres puis, toujours avec la même commande de rotation de l'arbre (ou des arbres) qui supporte(nt) la (ou les) lame(s), c'est en second lieu l'autre seconde portion de lame 23 qui pénètre dans l'os cortical de l'autre vertèbre.

Du fait du déphasage entre les pénétrations des deux portions de lame(s) dans les vertèbres, l'effort (ou selon les techniciens : le « couple de forces ») que le Praticien est obligé de fournir est nettement inférieur à celui qu'il aurait dû appliquer si les deux portions de lames avaient pénétré simultanément dans l'os cortical. Cet avantage contribue incontestablement à faciliter l'implantation d'une telle cage intersomatique, et donc le travail du Praticien, et ce pour le bien du patient.

Le disque 10 peut être réalisé par exemple en Titane, en Peek (i.e. polyétheréthercétone) ou analogue, et quant aux lames et arbres ils sont réalisés par exemple en Titane ou acier inoxydable ou analogue.

Il est aussi bien stipulé qu'une telle cage peut comporter une ou plusieurs lames, même si elle n'en comporte habituellement que deux (figure 3).

Par « portion de lame » au sens de la présente description, il doit être compris une des extrémités d'une telle lame. Mais il est possible qu'une telle portion soit constituée par exemple par au moins une dent ou analogue située latéralement sur la tranche de la lame, ou même par une combinaison de l'extrémité de la lame et d'une dent.

## Revendications

1. Cage intersomatique apte à être interposée entre deux corps de vertèbres en remplacement du disque intervertébral originellement situé entre ces deux vertèbres, ladite cage comportant :
• un élément présentant la forme générale d'un disque (10) comportant deux faces opposées (11, 12) sensiblement parallèles et une paroi latérale (13) reliant les deux dites faces opposées, les deux dites faces opposées étant aptes à venir au contact respectivement des deux corps vertébraux, ces deux faces opposées définissant sensiblement deux plans faciaux contenant ces deux faces,
• des moyens de blocage du dit élément (10) avec au moins l'un des deux corps vertébraux, ces moyens de blocage comportant au moins une première portion de lame (21) et une seconde portion de lame (22), et
des moyens pour commander en déplacement des première et seconde portions de lame entre deux positions, la première position (Pr) étant celle dans laquelle les première et seconde portions de lame (21, 22) sont entièrement contenues dans l'espace défini entre les deux plans faciaux, et la seconde position (Ps) étant celle dans laquelle ces première et seconde portions de lame (21, 22) émergent du dit espace, ces des première et seconde portions de lame entre deux positions étant agencés pour commander le déplacement de ces première et seconde portions de lame selon un déphasage non nul de façon que l'une des première et seconde portions de lame émerge dudit espace avant l'autre portion de lame, **caractérisée par le fait que** les première et seconde portions de lame (21, 22) sont définies sur une seule lame (23, 24), ladite seule lame (21, 23) étant solidaire d'un arbre de rotation (123) d'axe de rotation (124) de façon que le plan de cette seule lame fasse, avec cedit axe de rotation, un angle non nul, ledit arbre de rotation étant monté en coopération par rapport à l'élément (10) de façon que son axe de rotation soit sensiblement parallèle aux dites faces opposées (11, 12).

2. Cage selon la revendication 1, **caractérisée par le fait que** ledit angle non nul est un angle droit.

3. Cage selon la revendication 1, **caractérisée par le fait que** ladite seule lame (23) est une lame symétrique.

4. Cage selon la revendication 1, **caractérisée par le fait que** les deux portions de lame (21, 22) sont définies à chaque extrémité d'une seule lame asymétrique (24).

## Patentansprüche

1. Zwischenkörperkäfig, der geeignet ist, zwischen zwei Wirbelkörpern als Ersatz für die Bandscheibe eingesetzt zu werden, die sich ursprünglich zwischen diesen zwei Wirbeln befindet, der Käfig umfassend:
• ein Element, das die allgemeine Form einer Scheibe (10) aufweist, umfassend zwei gegenüberliegende Flächen (11, 12), die im Wesentlichen parallel sind, und eine Seitenwand (13), die die zwei gegenüberliegenden Flächen verbindet, wobei die zwei gegenüberliegenden Flächen geeignet sind, jeweils mit den zwei Wirbelkörpern in Kontakt zu kommen, wobei diese zwei gegenüberliegenden Flächen im Wesentlichen zwei Gesichtsebenen definieren, die diese zwei Flächen enthalten,
• Blockierungsmittel für das Element (10) mit mindestens einem der zwei Wirbelkörper, diese Blockierungsmittel umfassend mindestens einen ersten Klingenabschnitt (21) und einen zweiten Klingenabschnitt (22), und
Mittel zum Steuern einer Bewegung des ersten und des zweiten Klingenabschnitts zwischen zwei Positionen, wobei die erste Position (Pr) diejenige ist, in der der erste und der zweite Klingenabschnitt (21, 22) vollständig in dem Raum enthalten sind, der zwischen den zwei Gesichtsebenen definiert ist, und die zweite Position (Ps) diejenige ist, in der dieser erste und dieser zweite Klingenabschnitt (21, 22) aus dem Raum hervorragen, wobei dieser erste und dieser zweite Klingenabschnitt zwischen zwei Positionen zum Steuern der Bewegung dieses ersten und dieses zweiten Klingenabschnitts gemäß einer von null verschiedenen Phasenverschiebung angeordnet sind, so dass einer des ersten und des zweiten Klingenabschnitts vor dem anderen Klingenabschnitt aus dem Raum hervorragt, **dadurch gekennzeichnet, dass** der erste und der zweite Klingenabschnitt (21, 22) auf einer einzigen Klinge (23, 24) definiert sind, wobei die einzige Klinge (21, 23) mit einer Drehwelle (123) mit Drehachse (124) derart fest verbunden ist, dass die Ebene dieser einzigen Klinge mit der Drehachse einen von null verschiedenen Winkel ausbildet, wobei die Drehwelle in Zusammenwirkung relativ zu dem Element (10) derart montiert ist, dass ihre Drehachse im Wesentlichen parallel zu den gegenüberliegenden Flächen (11, 12) ist.

2. Käfig nach Anspruch 1, **dadurch gekennzeichnet, dass** der von null verschiedene Winkel ein rechter Winkel ist.

3. Käfig nach Anspruch 1, **dadurch gekennzeichnet, dass** die einzige Klinge (23) eine symmetrische Klinge ist.

4. Käfig nach Anspruch 1, **dadurch gekennzeichnet, dass** die zwei Klingenabschnitte (21, 22) an jedem Ende einer einzigen asymmetrischen Klinge (24) definiert sind.

## Claims

1. Interbody cage suitable for being positioned between two vertebral bodies to replace an intervertebral disk originally located between these two vertebrae, said cage comprising:
• an element in the general shape of a disk (10), having two substantially parallel opposing faces (11, 12) and a side wall (13) connecting said two opposing faces, said two opposing faces being suitable for respectively coming into contact with the two vertebral bodies, said two opposing faces substantially defining two facial planes containing said two faces,
• means for locking said element (10) to at least one of the two vertebral bodies, said locking means comprising at least a first blade portion (21) and a second blade portion (22), and
means for controlling movement of the first blade portion and the second blade portion between two positions, the first position (Pr) being the position in which the first blade portion and the second blade portion (21, 22) are entirely contained in the space defined between the two facial planes, and the second position (Ps) being the position in which said first blade portion and said second blade portion (21, 22) emerge from said space, said the first blade portion and the second blade portion between two positions being arranged to control the movement of said first blade portion and said second blade portion according to a non-zero phase shift such that one of the first blade portion and the second blade portion emerges from said space before the other blade portion,
**characterized in that** the first blade portion and the second blade portion (21, 22) are defined on a single blade (23, 24), said single blade (21, 23) being rigidly connected to a rotating shaft (123) having a rotational axis (124) such that the plane of said single blade forms a non-zero angle with said rotational axis, said rotating shaft being mounted in engagement with respect to the element (10) such that the rotational axis of said rotating shaft is substantially parallel to said opposing faces (11, 12).

2. Cage according to claim 1, **characterized in that** said non-zero angle is a right angle.

3. Cage according to claim 1, **characterized in that** said single blade (23) is a symmetrical blade.

4. Cage according to claim 1, **characterized in that** the two blade portions (21, 22) are defined at each end of a single asymmetrical blade (24).
